# EUROPÄISCHE PATENTSCHRIFT

(11) **EP 1 423 377 B1**
(45) Veröffentlichungstag und Bekanntmachung des Hinweises auf die Patenterteilung: **19.01.2005**
(21) Anmeldenummer: 02797933.5
(22) Anmeldetag: 29.08.2002
(51) Int. Cl.: C07D 317/36

(54) **VERFAHREN ZUR HERSTELLUNG VON GLYCERINCARBONAT**
METHOD FOR PRODUCING GLYCEROL CARBONATE
PROCEDE DE PRODUCTION DE CARBONATE DE GLYCEROL

(30) Priorität: 07.09.2001 DE 10143973
(43) Veröffentlichungstag der Anmeldung: 02.06.2004
(73) Patentinhaber: Cognis Deutschland GmbH & Co. KG, 40589 Düsseldorf (DE)
(72) Erfinder: HERAULT, David, F-77780 Bourron-Marlotte (FR); BOUTTY, Bernd, 40667 Meerbusch (DE); ZANDER, Lars, 41569 Rommerskirchen (DE); STRUBE, Albert, 41470 Neuss (DE)
(86) Internationale Anmeldenummer: PCT/EP2002/009631
(87) Internationale Veröffentlichungsnummer: WO 2003/022829

(56) Entgegenhaltungen:
- EP-A- 0 739 888
- WO-A-01/66510
- US-A- 2 766 258
- US-A- 2 894 957
- US-A- 2 915 529
- DATABASE WPI Derwent Publications Ltd., London, GB; AN 2001-605177[69] XP002223452 HIROTSU KENJIKANEKO TAKAYOSHI: "Method for producing 4-hydromethyl-1,3-dioxolan-2-one" & JP 2001 172277 A (UBE IND LTD), 21. Dezember 1999 (1999-12-21)

## Beschreibung

Die vorliegende Erfindung betrifft ein Verfahren zur Herstellung von hochreinem Glycerincarbonat.

Bei der Herstellung von Glycerincarbonat (systematisch 4-Hydroxymethyl-1,3-Dioxolan-2-on) entstehen neben dem gewünschten Hauptprodukt als Nebenprodukt immer auch oligomere Produkte, vorzugsweise Diglycerincarbonat. Weiterhin verbleibt ein Teil des unreagierten Glycerins im Reaktionsprodukt. Je nach Einsatzgebiet des Glycerincarbonats sind diese Nebenprodukte störend und müssen ggf. durch aufwendige Reinigungsverfahren wie Hochvakuumdestillation oder chromatographische Verfahren, aus dem Rohprodukt entfernt werden. Bislang ist im Stand der Technik kein Verfahren bekannt, welches Glycerincarbonat in praktisch 100-%iger Reinheit liefert.

Es besteht daher Bedarf, ein einfaches und preiswertes Verfahren zu finden, welches es ermöglicht, Glycerincarbonate in möglichst hoher Reinheit herzustellen. Es wurde nun gefunden, dass durch eine Nachbehandlung des Rohglycerincarbonats die Reinheit des Endprodukt gesteigert werden kann.

Gegenstand der vorliegenden Erfindung ist daher ein Verfahren zur Herstellung von hochreinem Glycerincarbonat, welches Glycerin und oligomeres Glycerincarbonat in Mengen von insgesamt höchstens 5 Gew.-% enthält, wobei man das rohe Glycerincarbonat in Gegenwart einer wasserfreien Base umsetzt und anschließend aufarbeitet.

Das erfindungsgemäße Verfahren geht zunächst aus von Rohglycerincarbonat, welches nach allen dem Fachmann bekannten Herstellungsverfahren gewonnen werden kann. Beispielsweise beschreibt die EP 955 298 ein Verfahren zur Herstellung Glycerincarbonaten durch direkte Umsetzung von Glycerin in überkritischem CO₂ in Gegenwart von Zeolithen und Ionenaustauschharzen. Aus der EP 739 888 ist ein Verfahren zur Herstellung von Glycerincarbonat bekannt, wobei Glycerin mit Ethylen- oder Propylencarbonaten in Gegenwart geeigneter Katalysatoren wie Zeolithen zur Reaktion gebracht wird. Auch die EP 582 201 beschreibt ein Verfahren zur Herstellung von Glycerincarbonat, wobei Glycerin mit CO und Sauerstoff bei erhöhter Temperatur in Gegenwart von Übergangsmetallkatalysatoren erzeugt wird. Besonders bevorzugt ist es, Rohglycerincarbonat einzusetzen, welches gemäß der Lehre der JP 95094450 erhalten wird.

Im Rahmen des vorliegenden erfindungsgemäßen Verfahrens wird Rohglycerincarbonat, welches in der Regel 3 bis 10 Gew.-% der oligomeren Glycerincarbonate und etwa 3 bis 10 Gew.-% freies Glycerin enthält, mit geeigneten wasserfreien Basen umgesetzt. Im Rahmen dieser Reaktion wird das vorhandene Rohglycerin bzw. das oligomere Glycerincarbonat substantiell verringert. Wesentlich ist dabei, dass die verwendeten Basen zum einen starke Basen sind, d. h. einen pk_{B}- Wert von maximal 5 aufweisen und gleichzeitig wasserfrei sind, d. h. sie enthalten kein Wasser oder max. 2 bis 5 Gew.-% Wasser, bezogen auf die Menge an Base.

Besonders bevorzugt im Rahmen der vorliegenden Erfindung ist die Verwendung von Basen, ausgewählt aus der Gruppe der tertiären Amine, der an Festphasen gebundenen Amine, der Alkali- und Erdalkalisilikate, vorzugsweise der Natriumsilikate, der Hydroxide der Alkaliund Erdalkalimetalle, der Carbonate und Hydrogencarbonate von Natrium, Kalium, Lithium und Calcium sowie von festen Salzen des Natriums, Kaliums Lithiums und Calciums von Alkylalkoholen. Besonders bevorzugt ist der Einsatz von Natriummetasilikaten, Calciumhydroxid oder von Triethylamin.

Bei den Natriumsilikaten handelt es ich um die Natriumsalze der verschiedenen Kieselsäuren. Man unterscheidet Natriumsilikate, bei denen das Verhältnis SiO₂/Na₂O ≥ 2 oder ≤ 1 ist. Zur letzteren Gruppe gehört das Natriummetasilicat (Na₂SiO₃), MG 122,06, das im vorliegenden Verfahren vorzugsweise eingesetzt wird. Es muss allerdings das wasserfrei Natriummetasilikat ausgewählt werden (Schmelzpunkt 1089 °C) und nicht die hydratisierten Formen. Besonders geeignet sind solche Basen, die selbst in Glycerincarbonat nicht oder nur gering löslich sind, da dann die Aufarbeitung des Endprodukts einfach durch Filtration erfolgen kann.

Die Basen werden im erfindungsgemäßen Verfahren in Mengen von 0,5 bis 12 Gew.-%, vorzugsweise von 1 bis 10 Gew.-% und insbesondere von 2 bis 5 Gew.-%, jeweils bezogen auf das rohe Glycerincarbonat, eingesetzt. Dabei setzt die gewünschte Reaktion bereits bei Raumtemperatur in ausreichendem Maße ein. Die Temperatur selber ist aber kein kritischer Faktor, so kann das erfindungsgemäße Verfahren auch bei niedrigeren oder höheren Temperaturen durch geführt werden. Vorzugsweise wird es aber bei Raumtemperatur, also 21 °C ausgeführt.

Die Reaktion ist bei 21°C in der Regel nach 30 bis 60 Min. beendet. Anschließend hat es sich als vorteilhaft erwiesen, das Reaktionsgemisch zunächst zu filtrieren und anschließend mit entsprechenden starken Säuren, vorzugsweise mit Phosphorsäure, auf einen pH-Wert von 4 bis 5 einzustellen. Anschließend kann es vorteilhaft sein, nochmals zu filtrieren, um das dann erhaltene reine Glycerincarbonat zu erhalten.

Das erfindungsgemäße Verfahren führt zu Glycerincarbonaten mit niedrigen Anteilen an freiem Glycerin und polymeren Glycerincarbonat. Vorzugsweise enthält das so gewonnene Glycerincarbonat mind. 94 Gew.-% Glycerincarbonat und insbesondere 95 Gew.-% Glycerincarbonat und mehr.

Das so gewonnene Glycerincarbonat eignet sich zum Einsatz in verschiedenen Anwendungsbereichen, wobei beispielsweise die Verwendung in Waschmitteln, Wasch- und Reinigungsmitteln oder der besonders bevorzugte Einsatz von hochreinem Glycerincarbonat als Feuchthaltemittel in kosmetischen Mitteln erwähnt sei.

### Beispiele

100 g rohes Glycerincarbonat, enthaltend nach HPLC-Analyse 5,4 Gew.-% Glycerin, 90,9 Gew.-% Glycerincarbonat und 3,7 Gew.-% oligomeres Glycerincarbonat, wurden jeweils für 1 Stunde mit verschiedenen Basen gemäß Tabelle 1 bei 21°C zur Reaktion gebracht. Anschließend wurde abfiltriert, der pH-Wert mit Phosphorsäure auf 4 bis 5 eingestellt und erneut filtriert. Die Ergebnisse der Reaktion sind ebenfalls in Tabelle 1 zusammengefasst (Ergebnisse von HPLC-Untersuchungen der Produkte):

**Tabelle 1**

| Ansatz | Base | Menge Gew.-% | Glycerincarbonat Gew.-% | Glycerin Gew.-% | Oligomer Gew.-% |
|---|---|---|---|---|---|
| A | - | - | 90,9 | 5,4 | 3,7 |
| B | NaOH conc. | 2 | 91,4 | 7,1 | 1,5 |
| C | Ca(OH)₂ | 10 | 94,2 | 4,1 | 1,7 |
| D | Simet AP | 5 | 95,5 | 3,5 | 1,0 |
| E | Simet AP | 10 | 95,2 | 4,0 | 0,8 |
| F | Triethylamin | 5 | 95,4 | 4,0 | 0,6 |

Bei Verwendung der erfindungsgemäßen Basen C - F wird das Glycerincarbonat in höherer Reinheit als bei Verfahren nach dem Stand der Technik (Ansätze A und B) erhalten.

## Patentansprüche

1. Verfahren zur Herstellung von hochreinem Glycerincarbonat, enthaltend Glycerin und oligomeres Glycerincarbonat in Mengen von insgesamt höchstens 5 Gew.-% , **dadurch gekennzeichnet, daß** man rohes Glycerincarbonat in Gegenwart einer wasserfreien Base umsetzt und anschließend aufarbeitet.

2. Verfahren nach Anspruch 1, **dadurch gekennzeichnet, daß** die Base ausgewählt ist aus der Gruppe tertiäre Amine, an Festphasen gebundene Amine, Natriummetasilikate, Hydroxide der Alkali- und Erdalkalimetalle, Carbonate und Hydrogencarbonate von Natrium, Kalium Lithium und Calzium, feste Salze des Natriums, Kaliums, Lithiums und Calciums von Alkylalkoholen.

3. Verfahren nach den Ansprüchen 1 bis 2, **dadurch gekennzeichnet, daß** man Natriummetasilikate als Base einsetzt.

4. Verfahren nach den Ansprüchen 1 bis 2, **dadurch gekennzeichnet, daß** man Calciumhydroxid als Base einsetzt.

5. Verfahren nach den Ansprüchen 1 bis 2, **dadurch gekennzeichnet, daß** man Triethylamin als Base einsetzt.

6. Verfahren nach den Ansprüchen 1 bis 5, **dadurch gekennzeichnet, daß** man Basen auswählt, deren pK_{B}-Wert maximal 5 beträgt.

7. Verfahren nach den Ansprüchen 1 bis 5, **dadurch gekennzeichnet, daß** man die Basen in Mengen von 0,5 bis 12 Gew.-%, vorzugsweise von 1 bis 10 Gew.-% und insbesondere von 2 bis 5 Gew.-% jeweils bezogen auf das rohe Glycerincarbonat, einsetzt.

8. Verfahren nach den Ansprüchen 1 bis 6, **dadurch gekennzeichnet, daß** die Reaktion bei Raumtemperatur (21°C) durchgeführt wird.

## Claims

1. A process for the production of high-purity glycerol carbonate containing glycerol and oligomeric glycerol carbonate in total quantities of at most 5% by weight, **characterized in that** crude glycerol carbonate is reacted in the presence of a water-free base and then worked up.

2. A process as claimed in claim 1, **characterized in that** the base is selected from the group of tertiary amines, amines fixed to solid phases, sodium metasilicates, hydroxides of the alkali metals and alkaline earth metals, carbonates and hydrogen carbonates of sodium, potassium, lithium and calcium, solid salts of sodium, potassium, lithium and calcium of alkyl alcohols.

3. A process as claimed in claims 1 and 2, **characterized in that** sodium metasilicates are used as the base.

4. A process as claimed in claims 1 and 2, **characterized in that** calcium hydroxide is used as the base.

5. A process as claimed in claims 1 and 2, **characterized in that** triethylamine is used as the base.

6. A process as claimed in claims 1 to 5, **characterized in that** bases with a pK_{B} value of at most 5 are used.

7. A process as claimed in claims 1 to 5, **characterized in that** the bases are used in quantities of 0.5 to 12% by weight, preferably 1 to 10% by weight and more particularly 2 to 5% by weight, based on the crude glycerol carbonate.

8. A process as claimed in claims 1 to 6, **characterized in that** the reaction is carried out at room temperature (21 °C).

## Revendications

1. Procédé de préparation de carbonate de glycérine de haute pureté, contenant de la glycérine et du carbonate de glycérine oligomère en quantités d'au total au plus 5 % en poids,
**caractérisé en ce qu'**
on fait réagir du carbonate de glycérine brut en présence d'une base anhydre, puis on le purifie.

2. Procédé selon la revendication 1,
**caractérisé en ce que**
la base est choisie dans le groupe des amines tertiaires, des amines liées à des phases solides, des métasilicates de sodium, des hydroxydes des métaux alcalins et alcalino-terreux, des carbonates et hydrogéno carbonates de sodium, de potassium, de lithium et de calcium, des sels solides de sodium, de potassium, de lithium et de calcium d'alkyl alcools.

3. Procédé selon les revendications 1 à 2,
**caractérisé en ce qu'**
on utilise des métasilicates de sodium comme bases.

4. Procédé selon les revendications 1 à 2,
**caractérisé en ce qu'**
on utilise l'hydroxyde de calcium comme base.

5. Procédé selon les revendications 1 à 2,
**caractérisé en ce qu'**
on utilise la triéthylamine comme base.

6. Procédé selon les revendications 1 à 5,
**caractérisé en ce qu'**
on choisit des bases dont le pK_{B} est au maximum de 5.

7. Procédé selon les revendicatrions 1 à 5,
**caractérisé en ce qu'**
on utilise les bases en quantités de 0,5 à 12 % en poids, de préférence de 1 à 10 % en poids et en particulier de 2 à 5 % en poids, toujours par rapport au carbonate de glycérine brut.

8. Procédé selon les revendications 1 à 6,
**caractérisé en ce qu'**
on effectue la réaction à la température ambiante (21°C).
